# EUROPEAN PATENT APPLICATION

(11) **EP 1 780 285 A1**
(43) Date of publication of application: **02.05.2007**
(21) Application number: 06024897.8
(22) Date of filing: 30.07.2001
(51) Int. Cl.: C12N 15/86, C12N 15/51, C12N 15/33, C12N 7/04, A61K 39/29, C07K 14/02, A61K 39/295

(54) **Improved virus like particles (VLP) based on the small envelope protein of hepatitis B virus (HBsAg-S)**

(30) Priority: 31.07.2000 AU PQ912000
(62) Divisional of application: 01953691.1
(71) Applicant: Monash University, Victoria 3800 (AU)
(72) Inventor: Gowans, Eric James, Ashgrove QLD 4060 (AU); Macnaughton, Thomas Bernard, Burbank CA 91504 (US); Netter, Hans Jurgen, Herston QLD 4029 (AU)
(74) Representative: Turner, Rhiannon Rosalind

(57) **Abstract**

Isolated polynucleotides comprising a HBsAg-S coding sequence that is adapted to receive an insert coding sequence, within a part of the HBsAg-S coding sequence that encodes an exposed site within the external loop of HBsAg-S, and still encode a HBsAg-S that is able to assemble into a VLP. Proteins encoded by the polynucleotides, recombinant VLPs and various uses thereof are also described.

## Description

### FIELD OF THE INVENTION

The present invention relates to improved virus like particles, their nucleotide and protein coding sequences and various uses thereof.

### BACKGROUND ART

The small envelope protein (HBsAg-S) expressed by the hepatitis B virus (HBV) has the capacity to self-assemble with host-derived lipids into empty envelope particles without the participation of nucleocapsids. These distinct forms of subviral particles that are spherical or filamentous and 22nm in diameter bud into the lumen of a pre-Golgi compartment and are subsequently secreted.

During synthesis of these particles HBsAg-S is cotranslationally inserted into the membrane of the endoplasmic reticulum (ER) to result in a short luminal exposed N-terminal sequence, two transmembrane regions separated by a 57aa cytosolic loop, a luminal external 70aa domain containing the major B-cell epitopes (the 'a'-determinant) and a glycosylation site. The 'a'-determinant consists of a limited number of epitopes and is located within a double-looped structure between about amino acids 124 and 147 of HBsAg-S (other authors have identified the 'a' determinant as between amino acids 120 and 150). It is estimated that one 22nm particle contains about 100 HBsAg-S molecules. The subviral HBsAg particles are used successfully worldwide for hepatitis B vaccination.

The particulate nature of virus like particles (VLPs) generally induces a more effective immune response than denatured proteins or soluble proteins. VLPs have a number of advantages over conventional immunogens as vaccines. Antigens from various infectious agents can be synthesized as VLPs in heterologous expression systems. In addition to the ability of certain capsid or envelope proteins to self-assemble, these particles can be produced in large quantities, and are easily enriched and purified. Vaccination with chimeric VLPs can induce both insert-specific B and/or T-cell responses even in the absence of adjuvant; furthermore VLPs cannot replicate and are non-infectious.

HBsAg particles have been used to generate chimeric particles carrying foreign epitopes. However, the location of the inserts in the prior art chimeras does not allow an optimal surface orientation of the foreign insert and may also compromise the ability of the HBsAg to self assemble. Furthermore, the resulting VLPs may have a reduced secretion competence.

The present invention seeks to overcome the problems with the prior art HBsAg particles or at least provide an alternative by providing improved VLPs, their nucleotide and protein coding sequences and various methods of using the improved VLPs.

### DISCLOSURE OF THE INVENTION

The present invention provides an isolated polynucleotide comprising a HBsAg-S coding sequence that is adapted to receive an insert coding sequence, within a part of the HBsAg-S coding sequence that encodes an exposed site within the external loop of HBsAg-S, and still encode a HBsAg-S that is able to assemble into a VLP.

For the purposes of this invention the term "insert" is defined as a protein or portion thereof such as a polypeptide or peptide.

For the purposes of the present invention the term "HBsAg-S" when used in relation to a coding sequence includes all HBsAg-S sequences derived from any strain of HBV, such as but not limited to any strain of avihepadnaviruses and orthohepadnaviruses, such as but not limited to the human infecting HBV genotypes A-G, and serological groups ayw1, ayw2, ayw3, ayw4, ayr, adw2, adw4, adrq+ and adrq-.

For the purposes of the present invention it will be appreciated that there are conflicting reports on the start and finish of the external loop of HBsAg-S. Thus, the external loop may vary slightly and preferably is from amino acids 101 to 159; 101 to 163 or 99 to 169.

Furthermore, whilst reference is made herein to "HBsAg-S", persons skilled in the art understand that HBV encodes three envelope proteins: HBsAg-S, HBsAg-M and HBsAg-L that are related to each other. HBsAg-M consists of HBsAg-S and has a N terminal extension of 55 amino acids (the preS2-domain) and HBsAg-L consists of HBsAg-S, the preS2 domain and has a N terminal extension, depending on the subtype, of 108 or 119 amino acids (preS1-domain). Thus, for the purposes of the present invention it will be appreciated that reference herein to the HBsAg-S coding sequence includes HBsAg-S coding sequences that form part of HBsAg-M or HBsAg-L.

In addition, when reference is made herein to the external loop of HBsAg-S, a person skilled in the art will recognise that the precise location of this loop, in terms of amino acid positions, is reported differently in the literature. For instance, the external loop has been described as the region spanning amino acids 101 to 159; 101 to 163 and 99 to 169. Where reference is made herein to a particular amino acid region corresponding to the external loop it is to be understood that this also includes the other published amino acid regions corresponding to the external loop.

The HBsAg-S coding sequence may encode a HBsAg-S that can assemble into a secretion competent VLP. Secretion competent VLPs may be more conveniently isolated and purified. Alternatively, the HBsAg-S coding sequence may encode a HBsAg-S that assembles into a VLP that is retained within the host cell. Non secreted VLPs may be isolated using standard techniques apparent to those skilled in the art. For example, the VLPs may be isolated from cell lysates using chromatography or some other technique which selectively purifies the VLPs from the extraneous cellular matter.

The HBsAg-S coding sequence may be a native HBsAg-S polynucleotide that has been modified to render it capable of receiving the insert coding sequence. In this regard, the native HBsAg-S coding sequence may be adapted to receive an insert coding sequence within the part of the native HBsAg-S polynucleotide encoding:
(i) amino acids 114 to 160 or 169 of HBsAg-S; (ii) amino acids 120 to 160 of HBsAg-S (iii) the 'a' determinant of HBsAg-S; (iv) amino acids 120 to 150 of HBsAg-S; (v) amino acids 124 to 147 of HBsAg-S; (vi) amino acids 124-145,124-1.42, 124-140, 124-138, 124-136, 124-134, 124-132, 124-130, 124-128 or 124-126 of HBsAg-S; or (vii) amino acids 127 and 128 of HBsAg-S.

The particular location of the insert coding sequence may be varied by a person skilled in the art depending on the intended end use of the nucleic acid molecule of the invention. For example, when the insert coding sequence is within the part of the polynucleotide encoding the 'a' determinant of HBsAg-S and the encoded recombinant HBsAg-S is required to package hepatitis delta virus, the insert coding sequence should be adequately separated from the coding sequence for the glycosylation site within the 'a' determinant, to ensure the recombinant HBsAg-S can package hepatis delta virus. One example of the invention that fulfils these requirements is when the insert coding sequence is introduced between codons 127 and 128 of native HBsAg-S.

Thus, the present invention also provides an isolated polynucleotide comprising a native HBsAg-S coding sequence that is adapted to receive an insert coding sequence, between codons 127 and 128, and still encode a HBsAg-S that is able to assemble into a VLP.

Furthermore, to limit and preferably avoid the potential effects of anti-HBsAg antibodies (anti-HBs), it is preferable that the polynucleotides of the present invention encode a HBsAg-S protein that is adapted to receive an insert and not be unduly affected by antibodies to HBsAg. In this regard, preferably, the polynucleotides of the present invention are adapted to receive an insert coding sequence at a location that disrupts one or more epitopes of native HBsAg upon expression of the polynucleotide.

For the purposes of the present invention the term "disrupts" includes (i) the removal of the epitope such that any antibodies to the epitope are unable to bind to it and (ii) modification of the epitope such that any antibodies to the epitope are able to bind with reduced affinity.

Thus, the present invention also provides an isolated polynucleotide comprising a HBsAg-S coding sequence that is adapted to receive an insert coding sequence, within a part of the HBsAg-S coding sequence that encodes an exposed site within the external loop of HBsAg-S, and still encode a HBsAg-S that is able to assemble into a VLP and wherein the location of the insertion of the insert coding sequence is such that upon expression of the polynucleotide the expression product includes at least one disrupted epitope of native HBsAg.

In addition to the modifications made to render the native HBsAg-S coding sequence adapted to receive an insert coding sequence, other variations may be made to the native sequence to make it more suitable for a given task. Such additional changes a commonly referred to as "design features" and include the variation of codons to ensure optimal expression in a given host and other changes made to the sequence to assist the cloning of the sequence. Such additional changes are readily apparent to one skilled in the art.

For example, the HBsAg-S coding sequence may be a variant of the native HBsAg-S polynucleotide that is substantially different to the native sequence but, due to the degeneracy of the genetic code, still encodes HBsAg-S. Other variants include those that encode for mutant HBsAg-S with desired features or HBsAg-S with one or more conservative amino acid changes or polynucleotides that encode a shortened version of native HBsAg-S that retains its ability to assemble into a VLP.

HBsAg-S polynucleotides encoding shortened HBsAg-S may be routinely prepared by persons skilled in the art by removal of nucleotides encoding non-essential parts of HBsAg-S. Non-essential parts of the HBsAg-S sequence may be determined routinely by preparing deletion mutants and assessing their expression products for assembly into VLPs and or using computer models to determine the amino acids critical for tertiary structure and presentation of the insert upon expression. Preferably, the deletion mutants encode a protein capable of self-assembly into secretion competent VLPs. Particular, deletion mutants encode at least 75% of native HBsAg-S, more preferably at least 80% of the amino acids in native HBsAg-S, even more preferably at least 90% and still more preferably at least 95-99% of the amino acids of native HBsAg-S.

The insert coding sequence may be received in the HBsAg-S coding sequence in a number of ways apparent to one skilled in the art. The HBsAg-S coding sequence may be manipulated to encode a restriction site at which the insert coding sequence can be introduced.

Thus, the present invention also provides an isolated polynucleotide comprising a HBsAg-S coding sequence, which defines a restriction site within a part of the HBsAg-S coding sequence that encodes an exposed site within the external loop of HBsAg-S, and still encodes a HBsAg-S that is able to assemble into a VLP.

The particular restriction site engineered into the HBsAg-S coding sequence may be varied by a skilled person as necessary. Preferably, the inclusion of the restriction site doesn't affect the amino acid sequence of the encoded HBsAg-S. In one example, when the coding sequence is inserted between codon 127 and 128, the HBsAg-S coding sequence may be manipulated to encode a restriction site for AgeI that enables the HBsAg-S coding sequence to be cut and receive the insert coding sequence between codons 127 and 128. In particular, codon 127 in the native HBsAg-S coding sequence may be changed from ACT to ACC and codon 128 may be changed from GCT to GGT.

The size of the insert coding sequence may be varied, as required, to an upper limit at which the HBsAg-S coding sequence still encodes a HBsAg-S capable of assembly into a VLP. The insert coding sequence may encode up to about 5 to 100 amino acids, more particularly about 10 to 90 amino acids, about 20 to 80 amino acids, about 30 to 70 amino acids, or about 40 to 60 amino acids. In particular examples the insert coding sequence encodes about 35 or 60 amino acids.

The insert coding sequence may be of any origin including HBV, however, it will more often be of heterologous origin. Examples of insert coding sequences include bacterial, viral, animal and plant sequences. Particular viral sequences include HCV sequences, however, it will be appreciated that the insert coding sequence may be any sequence that encodes an insert that is useful when expressed as part of HBsAg-S. Such inserts include (i) immunological proteins or portions thereof that include an epitope and (ii) binding proteins or portions thereof that encode a binding domain.

In one form of the invention, when the insert coding sequence is a HCV sequence, the insert may encode an antigenic portion of HVR1 sequence of the E2 protein.

Preferably, expression of the polynucleotides of the present invention yields HBsAg-S that are able to assemble into a VLP upon which are presented the expression product of the insert coding sequence in a correct surface orientation.

For the purposes of the present invention the phrase "correct surface orientation" means that the conformation of the expressed insert is such that it retains at least one useful biological activity such as but.not limited to immunogenicity or binding capacity. Preferably, the correct surface orientation means that the expressed insert has a tertiary structure substantially similar to that of the insert when expressed in its native form.

The present invention also provides a method for producing an isolated polynucleotide encoding a HBsAg-S coding sequence comprising the steps of (i) isolating the HBsAg-S coding sequence (ii) modifying the HBsAg-S coding sequence such that, it is adapted to receive an insert coding sequence within a part of the HBsAg-S coding sequence that encodes an exposed site within the external loop of HBsAg-S, and still encode a HBsAg-S that is able to assemble into a VLP.

The present invention also relates to vectors that include the polynucleotides of the present invention, host cells which are genetically engineered with such vectors and the production of particles such as VLPs encoded by the polynucleotides.

The polynucleotides may be included in a vector, such as plasmid, containing a selectable marker for propagation. The vector may include an appropriate promoter such as the phage lambda PL promoter, the CMV promoter, the E. *coli* lac, trp and tac promoters, the SV40 early and late promoters and promoters of retroviral LTR's, to name a few. Other suitable promoters are readily apparent to one skilled in the art. Vectors designed to facilitate the expression of the polynucleotides of the present invention may further contain sites for transcription initiation, termination and in the transcribed region, a ribosome binding site for translation. The coding portion of the mature transcripts expressed by the constructs will preferably include a translation initiation at the beginning and a termination codon appropriately positioned at the end of the polypeptide to be translated.

The hosts cells comprising the polynucleotides of the present invention such as in the form of a vector may be varied as required and may be routinely selected by a person skilled in the art. Host cells may be of bacterial origin such as *E*. *coli*, fungal cells such as yeast; insect cells such as *Drosophila* and mammalian cells such as CHO, COS, cancer cell lines such as HuH-7.

The vector may be introduced into a host cell by any of a number of ways apparent to one skilled in the art including calcium phosphate transfection, DEAE-dextran mediated transfection, cationic lipid-mediated transfection, electroporation, transduction or infection.

The present invention also provides proteins, polypeptides and peptides encoded by any one of the polynucleotides of the invention described herein. These proteins, polypeptides and peptides may be assembled into virus like particles (VLPs) that are also part of the present invention.

Thus, the present invention also provides a virus like particle ("VLP") comprising a protein, polypeptide or peptide of the present invention.

One VLP of the present invention comprises a HBsAg-S and an insert located within the exposed site within the external loop of HBsAg-S, wherein said HBsAg-S is able to assemble into a VLP incorporating the insert.

Preferably, the VLPs of the present invention comprise a disrupted native epitope of HBsAg. In this regard, it is preferred that the location of the insert is such that native HBsAg epitope(s) in the region of the insert are disrupted.

Other VLPs according to the present invention comprise an assembly competent HBsAg-S and an insert within: (i) amino acids 114 to 160 or 169 of HBsAg-S; (ii) amino acids 120 to 160 of HBsAg-S (iii) the 'a' determinant of HBsAg-S; (iv) amino acids 120 to150 of HBsAg-S; (v) amino acids 124 to 147 of HBsAg-S; (vi) amino acids 124-145, 124-142, 124-140, 124-138, 124-136, 124-134, 124-132, 124-130, 124-128 or 124-126 of HBsAg-S; or (vii) amino acids 127 and 128 of HBsAg-S.

The VLPs of the present invention may be useful in various areas, the particular application being largely dependent on the biological activity of the insert. For example, the VLPs of the present invention may be capable of packaging hepatitis delta virus (HDV) and thus may be used to deliver HDV to a target cell.

Thus, the present invention also provides a HDV packaging VLP comprising a HBsAg-S and an insert located within the exposed site within the external loop of the HBsAg-S, wherein said HBsAg-S is able to assemble into a VLP Incorporating the insert. Preferably, the insert is located at or about amino acids 127 and 128 of HBsAg-S and more particularly between amino acids 127 and 128.

The size of the insert may be varied, as required, to an upper limit at which the VLP incorporating the insert is still assembly competent. Thus, the insert may encode between about 5 and 100 amino acids, more particularly between about 10 and 90 amino acids, between about 20 and 80 amino acids, between about 30 and 70 amino acids, or between about 40 and 60 amino acids. In particular examples the insert is up to about 35 or 60 amino acids.

The insert may be of any origin including HBV, however, the insert will more often be heterologous. Examples of inserts include those from bacteria, viruses, animals and plants. Particular viral inserts include HCV proteins sequences, however, it will be appreciated that the insert may be any molecule that is useful when expressed as part of HBsAg-S. Such inserts include (i) immunological proteins or portions thereof that include an epitope and (ii) binding proteins or portions thereof that encode a binding domain.

As indicated above the HBsAg-S protein of the present invention may also be adapted to package HDV. Thus, the present invention also provides a VLP of the present invention described herein comprising a HDV sequence.

Preferably, the VLPs of the present invention encode HBsAg-S that are adapted to form particles upon which are presented the insert in a correct surface orientation.

The present invention also provides a method of producing a VLP comprising the steps of: (i) transfecting a cell with a vector encoding a HBsAg-S and an insert and being capable of assembling into the VLP; (ii) culturing said cell under conditions that enable the expression of the HBsAg-S including the insert and the assembly into the VLP; and (iii) isolating the VLP.

As indicated above, the VLPs of the present invention have a number of applications limited only by the biological activity of the insert contained therein. Thus, the present invention also provides a pharmaceutical composition comprising a VLP of the present invention and physiologically acceptable carrier.

One use of the VLPs of the present invention is in the area of immunology. Thus, the present invention also provides a method of generating an immune response in a patient comprising the step of administering to said patient an effective amount of an immunogen including a VLP with an immunogenic insert according to the present invention.

It may be possible to invoke a potentiated immune response to the immunogenic preparations of the present invention by administering a HBV vaccine prior to administering the immunogenic preparation of the present invention. Thus, the present invention also provides a method of generating an immune response in a patient comprising the steps of (i) administering to said patient a HBV immunogenic preparation and (ii) administering to said patient an effective amount of an immunogen including a VLP with an immunogenic insert according to the present invention.

Furthermore, the present invention provides an immunogenic preparation comprising a VLP of the present invention. The VLPs in the immunogenic preparation may all contain the same immunogenic insert. Alternatively, the immunogenic preparation may contain a plurality of VLPs, each with a different immunogenic insert. Immunogenic preparations comprising a plurality of different VLPs are particularly useful when it is necessary to invoke a broad immune response. For example, when designing a viral vaccine that is required to impart protection against a range of viral quasipecies, it may be desirable to administer an immunogenic preparation that includes a "cocktail" of at least two VLPs with different immunogenic inserts.

When the VLPs of the present invention comprise a therapeutically active agent, they may also be used to treat disorders in a patient. Thus, the present invention also provides a method for treating a disease or disorder in a patient comprising administering to said patient an effective amount of VLPs of the present invention.

As an alternative to preparations comprising a plurality of different VLPs the present invention also provides for preparations comprising a VLP that expresses at least two different inserts. In this regard, hybrid VLPs may be assembled by different HBsAg-proteins, each containing a different insert such as a foreign epitope or binding protein.

VLP immunogenic preparations or "cocktails" may also be used to administer a single vaccine that invokes a protective or therapeutically beneficial immune response against a plurality of infectious agents.

The VLPs of the present invention may be used to selectively bind a target cell. In this regard, the VLPs may present a binding agent, such as a protein or portion thereof encoding a ligand, that enables the VLP to be targeted to cells that include a binding domain for the binding agent. The targeted VLPs may then be used to deliver agents such as therapeutics to a target cell.

Thus, the present invention also provides a method of delivering an agent to a target cell comprising the steps of (i) preparing a VLP which presents a binding agent for the target cell and (ii) contacting the VLP with the media containing the target cell.

The VLPs of the present invention may also present a therapeutic agent and thus the present invention also provides for a method of treating a disorder in an afflicted patient comprising the steps of administering to said patient an effective amount of a VLP presenting a therapeutic agent for said disorder.

The VLPs of the present invention may be used to produce HCV immunogenic preparations. Whilst the present invention is not limited to this application, it does include any of the above polynucleotides, proteins or portions thereof, VLPs and methods as applied to HCV immunogens and vaccines. Indeed, the examples section illustrates the use of the present invention as applied to HCV.

### BRIEF DESCRIPTION OF THE DRAWINGS

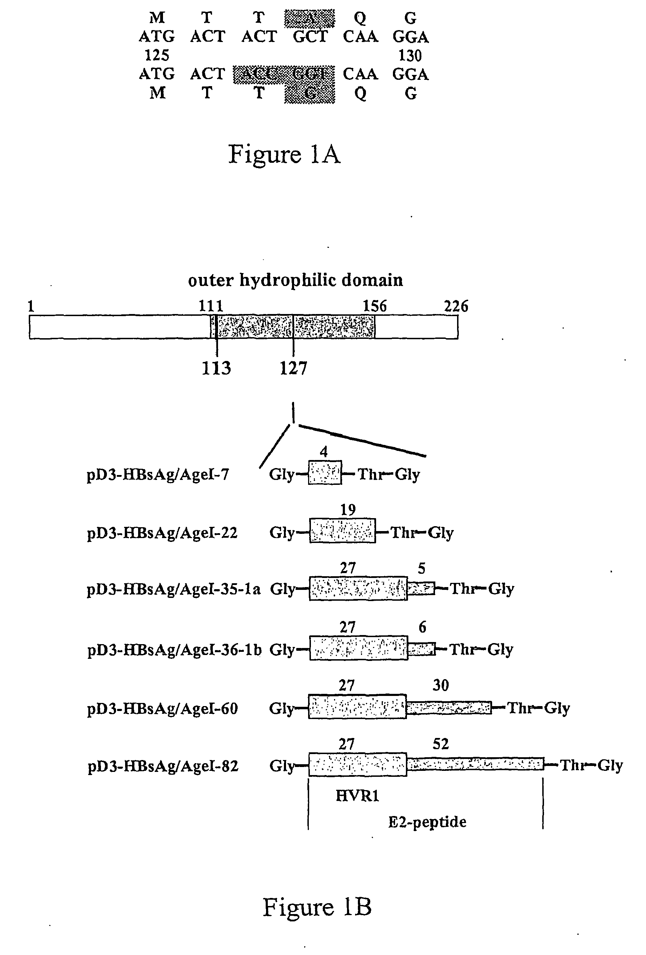

**Figure 1:** Illustration of the strategy to insert the HCV-HVR1 peptide into the hydrophilic loop of HBsAg-S. A) Part of the HBsAg-S nucleotide- and the corresponding amino acid-sequence before and after the introduction of the *Age*I cloning site. B) Constructs derived by inclusion of HCV sequences into the *Age*I cloning site of the modified HBsAg-S DNA sequence.

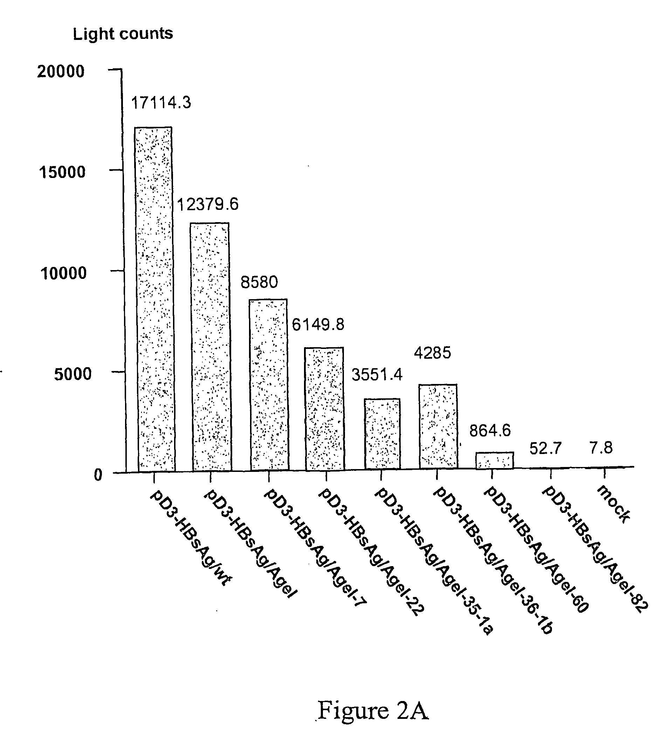

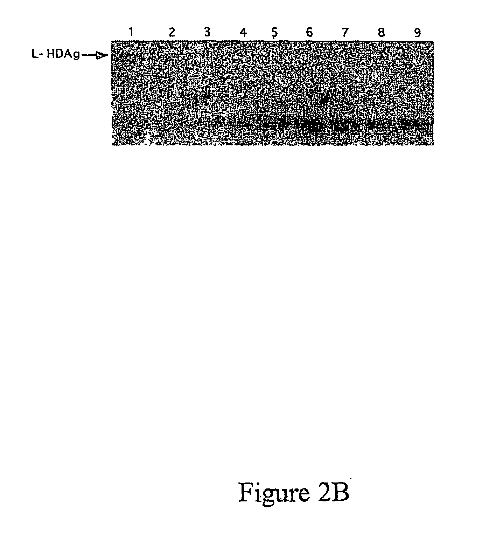

**Figure 2:** Detection of recombinant HBsAg and HDAg-L in cell culture supernatant. A) bar chart illustrating expression of recombinant HBsAg-proteins measured by chemiluminescence assay. B) immunoblot of HDAg-L secreted in the presence of the supernatants containing the recombinant HBsAg proteins - lane 1: HBsAg-wildtype, lane 2: HBsAg/AgeI, lane 3: HBsAg/AgeI-7, lane 4: HBsAg/Agel-22, lane 5: HBsAg/Agel-35-1a, lane 6: HBsAg/Agel-36-1b, lane 7: HBsAg/Agel-60, lane 8: HBsAg/Agel-82 and lane 9: no HBsAg.

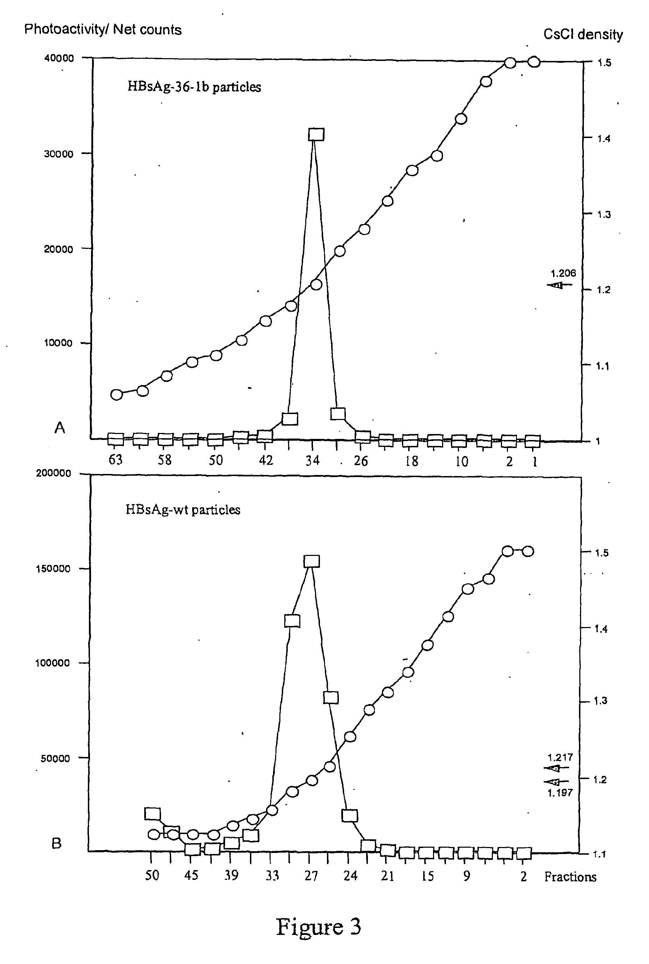

**Figure 3:** Equilibrium density gradient analysis of HBsAg-VLPs isolated from cell culture fluid A) Recombinant particles expressing HVR1-1b B) wildtype particles.

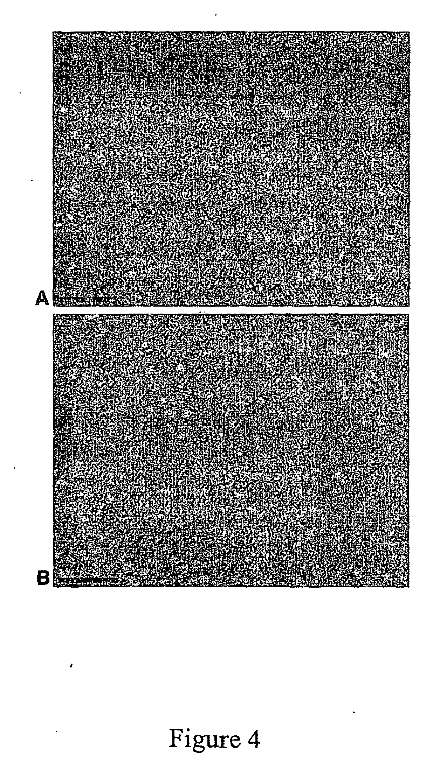

**Figure 4:** Identification of the particles by electron microscopy. A) wildtype HBsAg particles derived from carrier B) Recombinant particles derived from the construct pD3-HBsAg/Agel-36-1b.

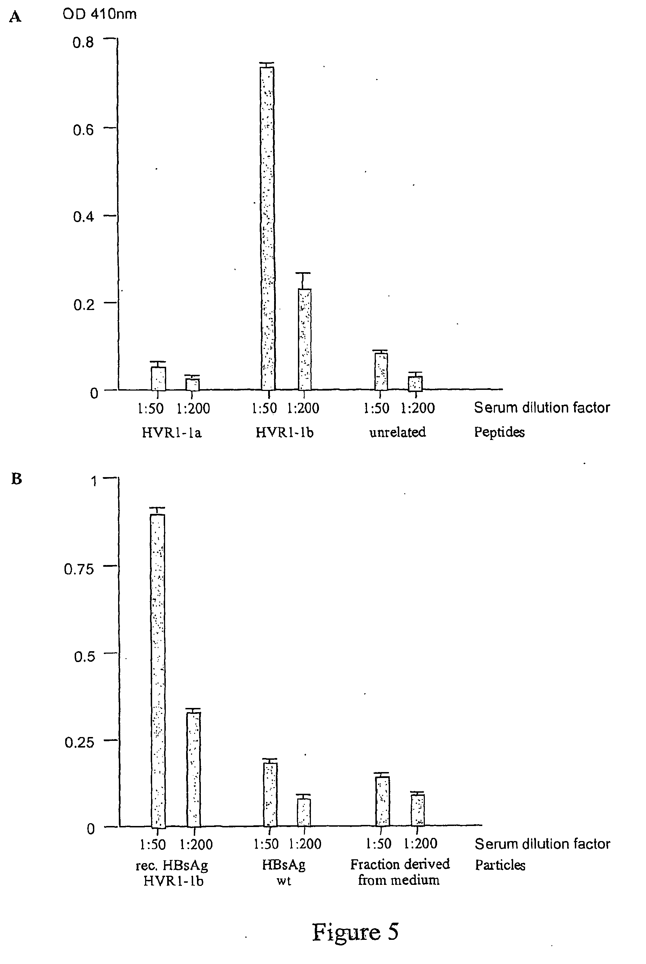

**Figure 5:** Reactivity of recombinant particles with human serum by ELISA. A) The serum from patient DK was tested against peptides representing HVR1-1band HVR-1a-sequences and an unrelated peptide. B) Serum DK was tested against recombinant HBsAg particles containing the HVR1-1b epitope, wildtype HBsAg particles, and a mock fraction derived from the cell culture fluid of untransfected HuH-7 cells.

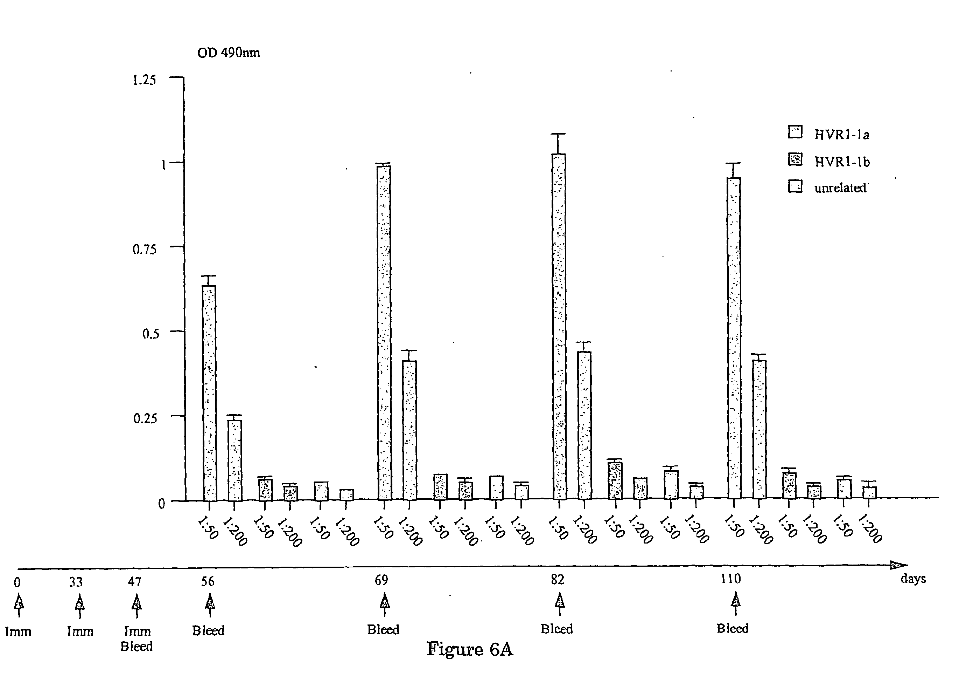

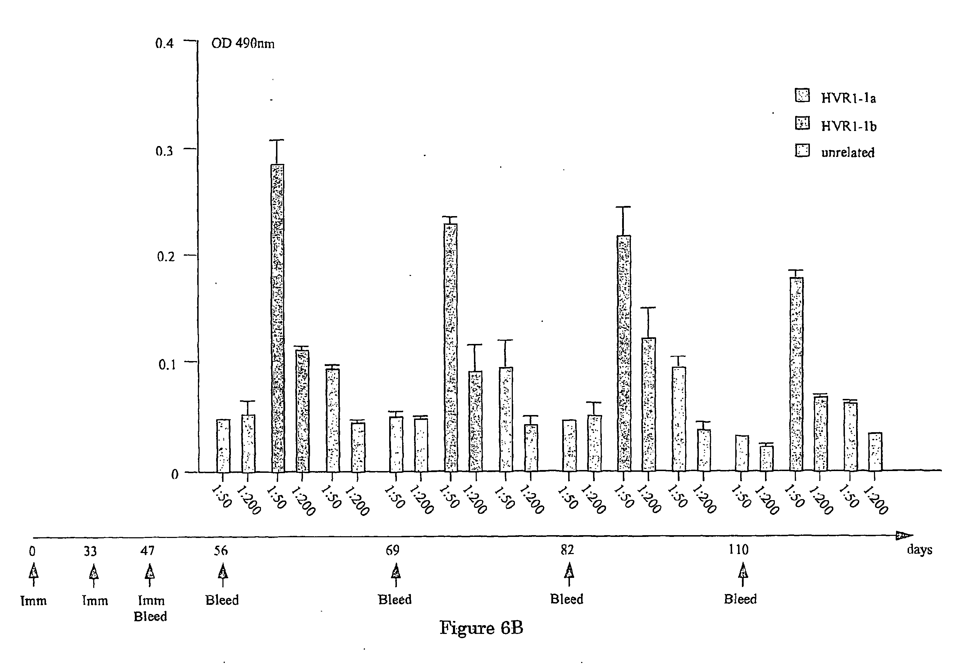

**Figure 6:** The immunogenicity of the recombinant VLPs in mice as determined by ELISA. Serum samples from a mouse immunized with HBsAg/Agel-35-1a recombinant particles (A) or serum samples from a mouse immunised with HBsAg/Agel-36-1b recombinant particles (B).

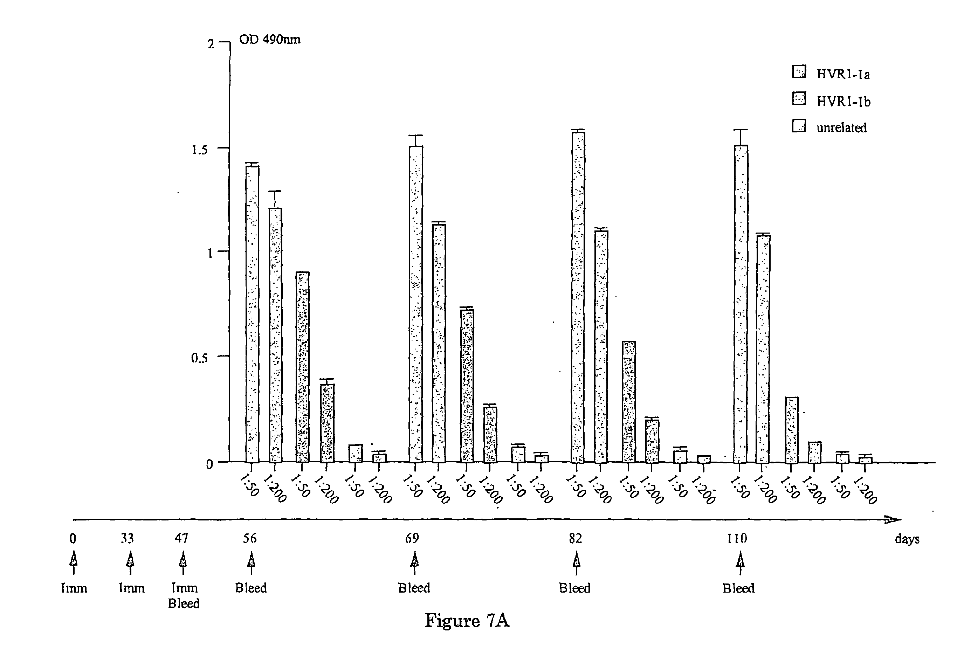

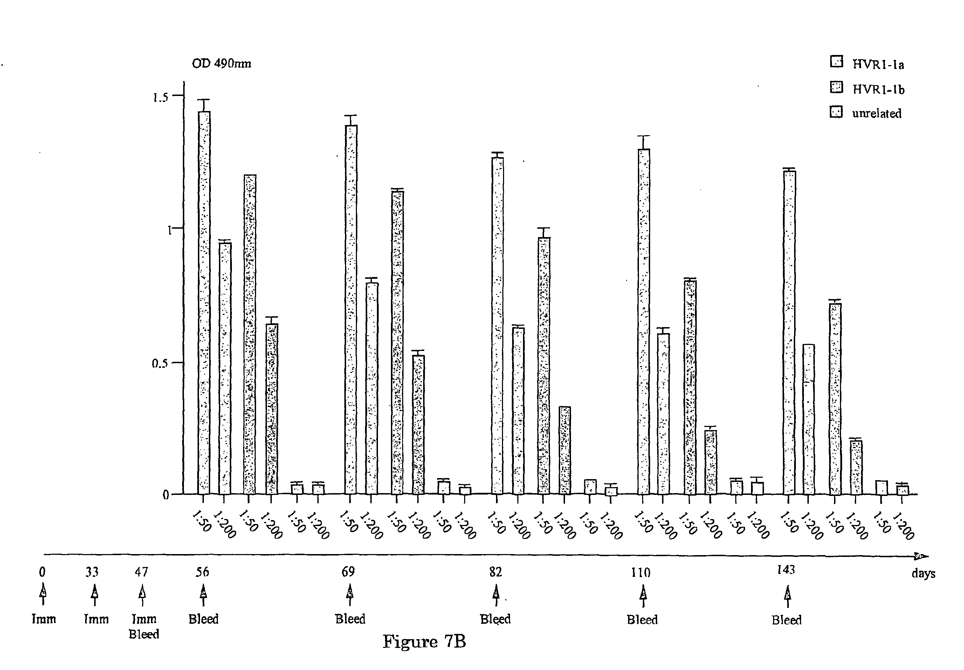

**Figure 7:** The induction of antibodies in mice immunized with a combination of VLPs as determined by ELISA. Sera were taken at different time points from two mice A) and B) immunized with HBsAg/Agel-36-1a and HBsAg/Agel-36-1b recombinant particles, respectively. Serum samples were tested against the HVR1-1a peptide, the HVR1-1b-specific peptide, and an unrelated peptide at 1:50 and 1:200 dilutions.

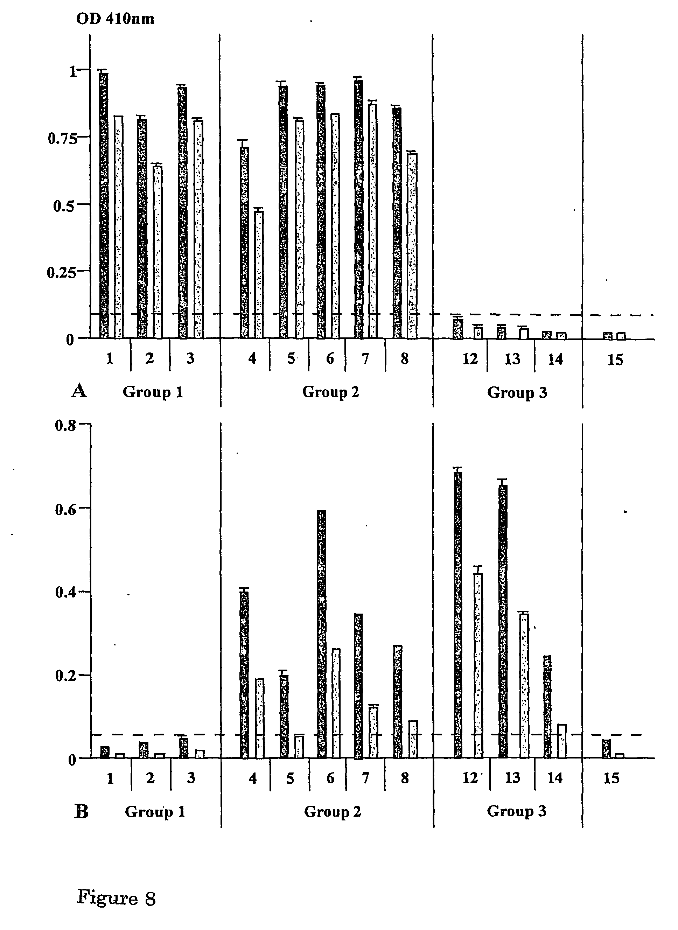

**Figure 8:** Immunogenicity of HBsAg particles in mice as determined by ELISA. A) Detection of anti-HBs; sera taken on day 33 were diluted 1:200 (black bar) and 1:400 (grey bar). B) Detection of anti-HVR1-1a; sera taken on day 75 were diluted 1:50 (black bar) and 1:200 (grey bar). The results show the mean OD of multiple tests and the standard deviation. A dashed line shows the cut-off value.

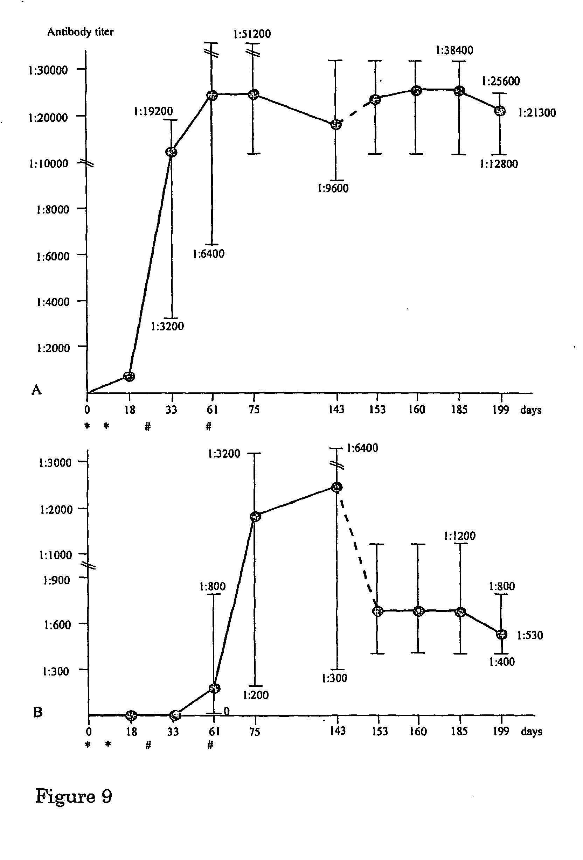

**Figure 9:** Antibody titer against HBsAg (A) and the HVR1-1a epitope (B) in serum samples from mice that were initially vaccinated with Engerix-B followed by vaccination with HBsAg/HVR1-1a VLPs (group 2, Table 2). The samples were taken at different time points, shown as a function of time (days). Asterisk identifies day 0 and 10 immunisations with Engerix-B, hash identifies day 24 and 61 immunisation with HBsAg/HVR1-1a VLPs. The titer is given as the arithmetic mean (shown by dots), and the bars indicate the highest and lowest antibody titer measured. The dashed line indicates that on day 143 two animals had to be sacrificed and from day 153 onwards the arithmetic mean of the titer derived from three animals is given.

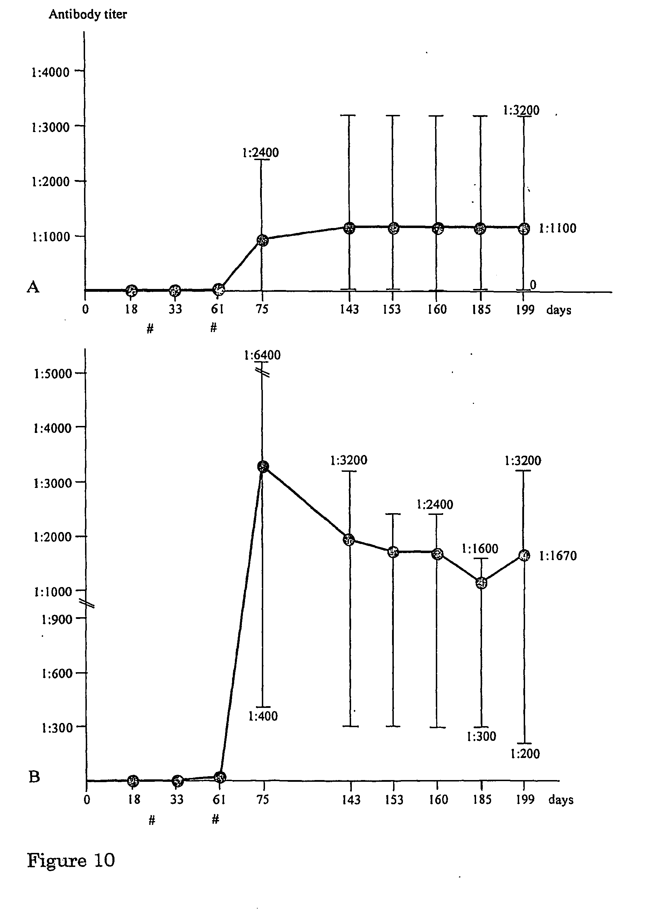

**Figure 10:** Antibody titer against HBsAg (A) and the HVR1-1a epitope (B) in mouse serum samples (group 3, Table 2) taken at different time points, shown as a function of time (days). Three mice were immunized on days 24 and 61 with HBsAg/HVR1-1a VLPs (indicated by an hash). The titer is given as the arithmetic mean (shown by dots), and the bars indicate the highest and lowest antibody titer measured.

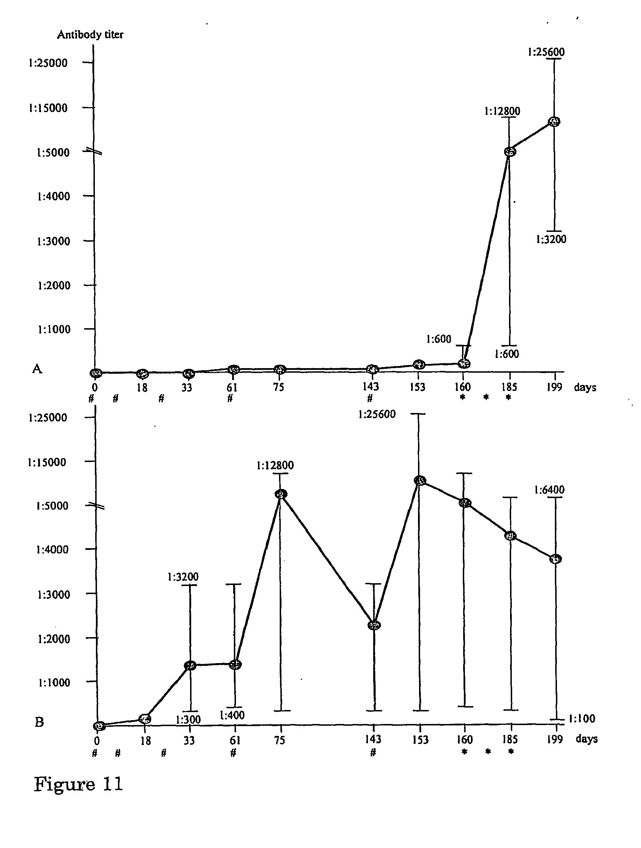

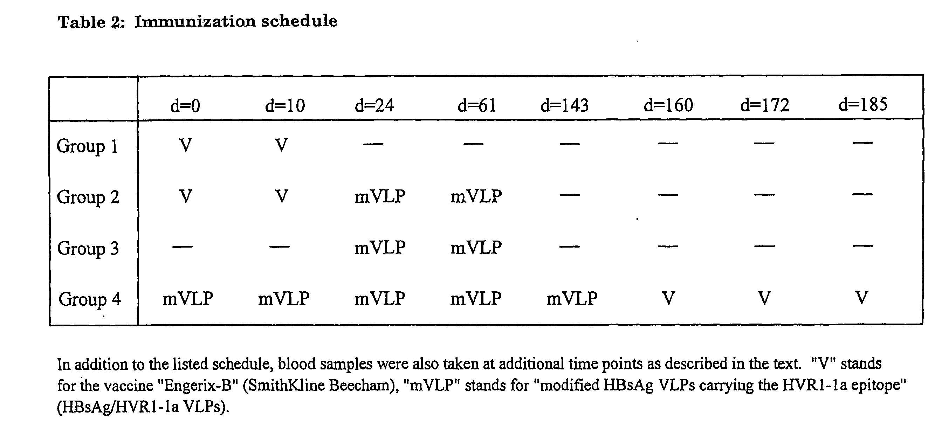

**Figure 11:** Antibody titer against HBsAg (A) and the HVR1-1a epitope (B) in serum samples from mice immunized with HBsAg/HVR1-1a VLPs followed by Engerix-B (group 4, Table 2). Three mice were immunized with HBsAg/HVR1-1a VLPs on days 0, 10, 24, 61 and 143 (indicated by the hash symbol) followed by immunizations with Engerix-B on days 160, 172 and 185 (indicated by an asterisk). The titer is given as the arithmetic mean (shown by dots), and the bars indicate the highest and lowest antibody titer measured.

### BEST MODE(S) FOR CARRYING OUT THE INVENTION

### General Materials and Methods

Plasmids: The plasmid pSVHBs (Harvey et *al*., 1997) was used as the template to amplify the gene for HBsAg-S. Two oligonucleotides (On#23 and On#24, Table 1) were designed to create an *Age*l-restriction site in the HBsAg-S cDNA which encodes for the 'a'-determinant region. The HBsAg-S-specific oligonucleotides On#24 and On#17 (Table 1) which anneal in the pSVL vector region upstream of the multiple cloning site were used to amplify the 5'-half of the HBsAg-S specific cDNA.

The HBsAg-S specific oligonucleotides On#23 and On#9 (Table 1) which anneal to the pSVL vector region downstream of the multiple cloning site, were used to amplify the 3'-half of the HBsAg-S specific cDNA. Both PCR-products contain an *EcoR*I restriction site outside the HBsAg-S ORF and an *Age*l restriction site within the HBsAg-S ORF.

To obtain the construct pD3-HBs/Agel both PCR fragments were digested with *EcoR*I and Agel, the vector pCDNA3 (Invitrogen) was digested with *EcoR*I and these DNA molecules were ligated. The ligation of the two fragments via the *Age*l site resulted in the restoration of the complete HBsAg-S ORF with an Agel restriction site at a position that corresponds to the amino acids 127/128 within the HBsAg-S. Due to the introduction of the Agel restriction site, the codon for amino acid 128-alanine of the wildtype HBsAg-S protein was mutated to glycine.

To clone the construct pD3-HBsAg/Agel-7, the oligonucleotides On#33 and On#34 (Table 1), were annealed and ligated into pD3-HBs/Agel via the Agel restriction site. The construct pD3-HBsAg/Agel-22 was generated in a similar manner by using On#35 and On#36 (Table 1). For the other constructs, HCV cDNA templates specific for the 1 b strain (Trowbridge and Gowans 1998) were used to amplify E2 specific products. The PCR-product was digested with *Age*l, and then inserted into D3-HBs/Agel.

For the construction of plasmid pD3-HBsAg/Agel-35-1a, a genotype HCV1a-cDNA template was used (Kolykhalov *et al*. 1997). The following sets of oligonucleotides were used; for pD3-HBsAg/Agel-35-1a: On#63 and On#64 (Table 1); for pD3-HBsAg/Agel-36-1b: On#62 and On#44 (Table 1); for pD3-HBsAg/Agel-60: On#62 and On#69; and for pD3-HBsAg/Agel-82: On#62 and On#70 (Table 1).

**Table 1: Sequence of oligonucleotides:**

| | |
|---|---|
| On#9 | 5'-GATGAATTCTCACTGCATTCTAGTTGTGG-3' |
| On#17: | 5'-GATGAATTCCTTCTGCTCTAAACCGGATCG-3' |
| On#23 | 5'-GACTACCGGTCAAGGAACCTCTATGTATCC-3' |
| On#24 | 5'-CTTGACCGGTAGTCATGCAGGTCCGGCATGG-3' |
| On#33 | 5'-CCGGTGGGGACACCCACACGA-3' |
| On#34 | 5'-CCGGTCGTGTGGGTGTCCCCA-3' |
| On#35 | 5'CCGGTGGGGACACCCACACGACGGGGGGGTGGCGG GCCGC GACACGCTGCGCTTCACGGGGTTCA-3' |
| On#36 | 5'-CCGGTGAACCCCGTGAAGCGCAGCGTGTCGCGGCCC GCCACCCCCCCCGTCGTGTGGGTGTCCCCA-3' |
| On#44 | 5'-TGACTACCGGTGGTGTTTACAAGCTGGATC-3' |
| On#62 | 5'-TGACTACCGGTGGGGACACCCACACGAC-3' |
| On#63 | 5'-TGACTACCGGTGGGGAAACCCACGTCACCGGG-3' |
| On#64 | 5'-TGACTACCGGTGTTGATCAGTTGGATG-3' |
| On#70 | 5'-GACTACCGGTGATGGGGTGGCAGCTGGC-3' |

Cell line and transfection: The human hepatoma cell line HuH-7 (Nakabayashi et al., 1982) was grown in DMEM medium (Gibco BRL) supplemented with GlutaMax-1 (Gibco BRL), 10% fetal calf serum, penicillin and streptomycin (Gibco BRL).

Transfection: HuH-7 cells were transfected by the Ca₃(PO₄)₂ method as described (Graham and van der Eb, 1973). The supernatant was harvested 5 days later, and HBsAg was measured by the Abbott Prism HBsAg assay (Abbott Diagnostics). The amount of HBsAg-S in the cell culture fluid was estimated by comparison with a commercially available vaccine (Engerix-B, 20µg/ml, SmithKline Beecham). The transfection efficiency of different plasmids was normalised for the activity of secreted alkaline phosphatase (SEAP) as described previously (Berger *et al*. 1988). The variation in the range of SEAP activity was less than threefold.

Peptides: The peptides representing the corresponding HVR1 regions of the HCV E2-protein, HVR1-1a and HVR1-1b, were synthesised at the Queensland Institute for Medical Research, Brisbane and had a purity of at least 50%.
- HCV HVR1-1a genotype: ETHVTGGSAGRTTAGLVGLLTPGAKQN
- HCV HVR1-1b genotype: DTHTTGGVAGRDTLRFTGFFSFGPKQK

An unrelated peptide was derived from the E7 protein of the human papilloma virus type 16. It was synthesized by Chiron Technologies, quality controlled by HLPC and mass spectroscopy.
- HPV-E7: DSTLRLCVQSTHVDIRTL

ELISA: Peptides (0.5µg/well) in PBS were bound to microtitre plates (Maxisorb, Nunc) at 4°C overnight, then each well was blocked in PBS plus gelatin (0.25%) and Tween20 (0.1%) at room temperature for 2h. The sera were incubated at an appropriate dilution in PBS plus gelatin (0.125%) and Tween20 (0.05%) for 1h at 37°C.

Antibody binding was detected by using an anti-mouse or anti-human immunoglobulin antibody conjugated to horseradish peroxidase (Dako). After several washing steps, antibody binding was visualised in the presence of ABTS (2.2'-Azino-bis (3-Ethylbenz-Thiazoline-6-sulfonic acid, Sigma) and H₂O₂ in a citrate phosphate buffer. Cell culture-derived VLPs expressing the HVR1-1 b peptide were purified over a sucrose cushion and by a CsCl gradient (see below).

In parallel, cell culture medium derived from untransfected HuH-7 cells was treated in the same way and mock fractions with the appropriate density were collected. The fractions were concentrated and the particles were purified from CsCl using a Microcon YM-100 filter device (Amicon). Each well was coated with about 500ng of VLPs in PBS as estimated by using the commercially available vaccine as standard. Patient serum sample was preincubated in cell culture medium to decrease the background signal, and then analysed by ELISA.

Human serum: The serum DK was derived from a patient from whom the Australian HCV isolate was cloned (Trowbridge and Gowans 1998).

Animals: C57Bl/6 and Balb/c mice were used at 6-15 weeks of age. Within a given experiment mice were littermates or were closely age- and sex-matched. The mice were housed under specific pathogen free conditions. Groups of two to four mice were immunized subcutaneously at the base of the tail with 250ng to 500ng of recombinant HBsAg VLPs in the presence of alhydrogel adjuvant. Mice used as negative controls were immunized with adjuvants alone. Mice were bled from the retro-orbital plexus at intervals commencing at 14 days after the second immunisation and the serum used in ELISA.

Centrifugation: Cell culture supernatant containing VLPs was overlaid on a 20% sucrose cushion (20% sucrose in STE-buffer: 100mM NaCl, 10mM Tris, pH8, and 1mM EDTA), centrifuged for 16h at 23,000 rpm (AH-629 rotor, Sorvall). The partially purified VLPs were resuspended in Hepes buffer and used for vaccination procedures.

To determine the buoyant density of the VLPs, and for purification purposes, the resuspended VLPs were loaded onto a 10% to 40% (w/w) CsCl step gradient (in STE-buffer), centrifuged for 22h at 36,000rpm (SW41Ti, Beckman), and 200µl fractions taken from the bottom of the tube. The fractions containing VLPs were identified by the Prism HBsAg assay (Abbott). The positive fractions were desalted, concentrated and washed with PBS by using Microcon YM-100 (Millipore) filter devices.

### Example 1 - HBsAg-S/HCV-HVR1 chimeric proteins

### Methodology/Results

We used the immunodominant HVR1-region as the foreign sequence to be inserted into the HBsAg-S subviral particles. To synthesise these VLPs we modified the HBsAg-S gene to create a new *Age*l restriction site that permitted insertion of the HVR1 into an exposed region of the major external hydrophilic loop of the 'a'-determinant. The construct was so designed to ensure a surface-orientation of the inserted HCV-specific B-cell epitope(s). The new *Age*I site within the HBsAg-S ORF led to an alanine to glycine change at position 128 (Fig. 1A). The numbers in Figure 1A indicate the amino acid position within HBsAg-S. The nucleotide sequence within the rectangle represents the Agel site.

A series of cDNA sequences encoding HCV-specific peptides of different lengths were inserted into the Agel site. Each insert starts with a glycine followed by the HVR1 sequence of the E2 protein derived from the Australian HCV-1b isolate (Trowbridge and Gowans 1998) and threonine and glycine at the C-terminal end of the insert. The different plasmids encoded 4aa, 19aa or the complete 27aa of the HVR1 region (Fig. 1B). Plasmids encoding the complete HVR1 region also contained the downstream 5aa, 6aa, 30aa or 52aa of the E2 protein as indicated (Fig. 1B). In addition, one construct was created (pD3-HBsAg/Agel-35-1a) which expressed the complete HVR1 polypeptide and the downstream 5aa derived from an HCV-1 a isolate (Kolykhalov *et al*. 1997).

In Figure 1 B the first amino acid is glycine which is not part of the sequence of the HCV-E2 protein, the last two amino acids (threonine and glycine) are encoded by the Agel nucleotide sequence downstream of the HCV-E2 insert. The stippled rectangle indicates the HVR1 region of E2 and the crosshatched rectangle represents the E2 sequence downstream of the HVR1 region. The numbers above the stippled and crosshatched rectangles indicate the number of the encoded amino acids of the corresponding HVR1-region and the downstream E2 region. The HBsAg-S sequence between amino acid 111 and 156 represents the outer hydrophilic domain.

### Example 2 - Detection of recombinant HBsAg and HDAg-L in cell culture supernatant

### Methodology/Results

HuH-7 cells were cotransfected with plasmids expressing HBsAg-proteins, HDAg-L and pSEAP. Supernatants were harvested and HBsAg measured by chemiluminescence assay (Figure 2A). The light counts were normalised by a SEAP assay. The identical supernatants were used to identify the secretion of HDAg-L in the presence of the different recombinant HBsAg proteins. 10ml of supernatant was pelleted through a sucrose cushion, resuspended in sample buffer, and analysed by an immunoblot specific for HDAg (Figure 2B).

The A128G mutation resulted in an apparent reduction in the level of secretion from pD3-HBsAg/Agel to approximately 70% compared with the wildtype HBsAg. Insertion of 7aa decreased the number of light counts to approximately 50%, while increasing the length of the HVR1 resulted in an even greater apparent reduction in secretion efficiency. As a result, the HBsAg which contained an insert of 82aa showed a similar number of light counts as the negative control (Fig. 2A). However the decreased number of light counts may reflect either a decreased secretion efficiency of the recombinant HBsAg proteins or reflect a decreased affinity of the anti-HBsAg IgM antibody (Prism HBsAg assay, Abbot Diagnostics) resulting from the modifications.

To address this question, cotransfections with a plasmid expressing the large hepatitis delta antigen (L-HDAg) were performed. L-HDAg can only be packaged and secreted in the presence of functional HBV envelope proteins, with HBsAg-S being sufficient for packaging (Chang *et al.* 1991, Wang *et al.* 1991). In this case, secretion was quantified by measurement of L-HDAg in the supernatant by immunoblot analysis (Fig. 2B).

The results of this experiment showed that recombinant HBsAg-S proteins containing an additional 35aa or 36aa in the external loop (Fig. 2B, lanes 5 and 6) were equally efficient as wildtype HBsAg-S (Fig. 2B, lane 1) to support L-HDAg secretion. Thus it is probable that these recombinant HBsAg-S proteins were secreted just as efficiently as wildtype HBsAg. Furthermore secretion of L-HDAg also indicates that these recombinant HBsAg-S proteins retained structural features necessary for this interaction.

On the other hand, co-expression with recombinant envelope proteins containing an insert of 60aa resulted in a decreased potential to support L-HDAg secretion (Fig. 2B, lane 7), and the recombinant protein with 82aa inserted into HBsAg-S was unable to support L-HDAg secretion (Fig. 2B, lane 8). These larger insertions may interfere with the stability of these proteins, the secretion capability of the recombinant HBsAg-S, and/or may lead to conformational changes which preclude L-HDAg secretion.

### Example 3 - Characterization of the recombinant particles.

### Methodology/Results

Although the above HBsAg preparations containing insertions of 35aa appeared to be secreted and recognized by the Prism HBsAg assay, it was important to determine if particle formation occurred. To this end, plasmids expressing wildtype HBsAg-S or the recombinant HBsAg/Agel-36-1b proteins were transfected independently into HuH-7 cells, the cell culture media collected and the particles concentrated and purified by centrifugation through a 20% sucrose cushion followed by a CsCl density gradient.

The HBsAg content of individual gradient fractions was measured by the Prism HBsAg assay. Wildtype- and recombinant-HBsAg were detected in fractions with density of 1.2 g/ml (Fig. 3). As this represents the density of wildtype HBsAg particles (Dubois *et al*. 1980, Moriarty *et al*. 1981) this provides strong evidence that the recombinant HBsAg formed particles in a similar manner to wildtype HBsAg.

To confirm this, the putative particles were examined by electron microscopy. Particles were derived from a HBV chronic carrier and from the supernatant of HuH-7 cells transfected with the plasmid expressing HBsAg/Agel-36-1b, then purified over a sucrose cushion and CsCl-gradients described above. Both samples contained particles of approximately 22nm (Fig. 4) and the particles derived from the recombinant HBsAg were virtually indistinguishable from wild type. Filaments and Dane particles were not present in the sample derived from the recombinant protein.

### Example 4 - Reactivity of the recombinant particles with human serum.

### Methodology/Results

The recombinant particles were then examined to determine if the HCV HVR1 region was displayed on the surface of the recombinant 22nm particles and whether its antigenicity is contained. As the Australian isolate of HCV (genotype 1b) was cloned from a single individual (DK) who acquired acute hepatitis C after receiving an allogeneic bone marrow transplant (Trowbridge and Gowans 1998), the serum from this patient was examined by ELISA for antibodies directed against the HVR1 region, initially using HVR1-specific peptides and later using the recombinant particles. HVR1-specific peptides were used that represented the sequence of the authentic Australian HCV1-1 b isolate and an HVR1-1a isolate (Kolykhalov et al. 1997).

The DK serum showed a specific reaction with the HVR1-1b peptide but did not react with the HVR1-1a peptide or an unrelated peptide (Fig. 5A). We then investigated if the HVR1-1b epitope presented by the VLPs had retained its antigenicity. The serum reacted exclusively with the VLPs with the expressed HVR1-1b epitope, but not wildtype VLPs, or the mock fraction (Fig. 5B). This indicated that the HVR1-epitope presented within the 'a'-determinant of HBsAg-S had retained its antigenicity and was most likely displayed on the surface of the VLP in a conformation identical or similar to the conformation of the HVR1 during natural infection.

### Example 5 - The recombinant VLPs are Immunogenic in mice.

### Methodology/Results

The particles expressed from plasmids HBsAg/Agel-35-1a or HBsAg/Agel-36-1 b (Fig. 1 B) were partially purified and injected into mice. In two experiments, four mice in total were immunized with the HVR1-1a particles and four mice were immunized with the HVR1-1b particles. As determined by ELISA, the sera of all four mice injected with the HBsAg-S/HVR1-1a particles were reactive with the HVR1-1a peptide. Serum taken on days 9 or 19 after the last booster injection showed an antibody titre against the HVR1-1a peptide that ranged 1:400 and 1:3200 (data not shown).

Representative results for one mouse immunized with HBsAg/Agel-35-1 a VLPs are shown in Fig. 6A. Sera were taken at different time points and tested (1:50 and 1:200 dilutions) against the HVR1-1a peptide, the HVR1-1b-specific peptide, and an unrelated peptide. The results show the mean of multiple tests and the standard deviation.

The serum sample taken at day 56 (9 days after the last booster injection) had an antibody titre of 1:1600 against the HVR1-1a peptide and this level of antibody persisted for at least 9 weeks. Similarly, the sera of three of four mice injected with the HBsAg-S/HVR1-1b particles were reactive with the HVR1-1b peptide. However, the antibody titres obtained against the HVR1-1b peptide were lower (range between 1:50 and 1:800, data not shown), suggesting that the HVR1-1b epitope was less immunogenic in the mouse system. Representative results for one mouse immunized with HBsAg/Agel-36-1b VLPs are shown in Fig. 6B. The antibody titre against the HVR1-1b peptide was 1:800 and this also persisted for at least 9 weeks.

Mice immunised with HBsAg/Agel-35-1a VLPs did not develop antibodies which were crossreactive with the HVR1-1b peptide, and *vice versa*. This is consistent with the above data from ELISA examination of the patient's serum which, although reactive with the HVR1-1b peptide, showed no crossreactivity with the HVR1-1a peptide (Fig. 5A).

### Example 6 - Immunisation with a combination of VLPs.

### Methodology/Results

To investigate whether antibodies could be raised simultaneously against the HVR1-1a and HVR1-1 b epitopes, four mice were immunised with an equimolar mix of HBsAg/Agel-35-1a VLPs and HBsAg/Agel-36-1b VLPs, and the antibody response against the individual peptides tested by ELISA.

Serum samples from three of four mice reacted with both epitopes, and the sample from the fourth mouse reacted weakly against the HVR1-1a epitope (titre: between 1:50 and 1:200) but not against the HVR1-1b epitope (data not shown). The serum samples of the three mice (taken at day 56, i.e. 9 days after the last booster injection) which responded strongly against both HVR1-1 a and -1b epitopes showed a titre against the HVR1-1a epitope ranging from 1:6400 to 1:12800. The antibody titre against the HVR1-1b epitope ranged from 1:1600 to 1:6400. In both instances, these titres were considerably higher than those generated by immunisation with the individual recombinant particles. Representative results from two mice are shown in Fig. 7.

The results suggest that a synergistic effect may account for the higher titres resulting from immunization with the mixed recombinant particles. The antibodies to peptides HVR1-1a and HVR1-1b persisted for at least twelve weeks after the last booster immunisation.

### Example 7 - Pre-existing immunity to HBV surface antigen permits revaccination with recombinant HBV-specific VLPs

### Objective

The aim of this example was to investigate whether pre-existing antibodies against HBsAg (anti-HBs) influence the generation of anti-HVR1 antibodies resulting from vaccination with recombinant HBsAg particles which contain the HVR1-1a sequence.

### Methodology/Results

The construct used in this example was plasmid pD3-HBsAg/Agel-35-1a and the hepatitis B vaccine used was Engerix-B.

The human hepatoma cell line HuH-7 (8) was grown in Dulbecco's modified Eagle's medium (Gibco-BRL) supplemented with Glutamax-1 (Gibco-BRL), 10% foetal calf serum, penicillin, and streptomycin (Gibco-BRL). HuH-7 cells were transfected by the Ca₃(PO₄)₂ method as described (4). The supernatant was harvested 5 days later, and the presence of HBsAg-S was measured by the Abbott Prism HBsAg assay (Abbott Diagnostics).

For immunization, the recombinant VLPs were partially purified and used in the presence of Alhydrogel adjuvant as described above.

Balb/c mice were used at 6 to 15 weeks of age and within an experiment were litter mates or were closely age and sex matched. The mice were kept under specific pathogen free conditions. For immunization with Engerix-B, (20ug/ml HBsAg, SmithKline Beecham), 100ul was injected subcutaneously into the base of the tail. About 500ng of recombinant VLPs were used for vaccination as described above. Mice.used as negative controls were immunized with adjuvants alone. Mice were bled from the retro orbital plexus or tail vein at intervals, and antibody levels measured by ELISA. The ELISA was performed as described above using microtiter plates coated with the HVR1-1a peptide (500ng/well) or yeast-derived HBsAg (50ng/well).

Four groups of Balb/c mice were examined; group 1 received the Engerix-B vaccine only, group 2 received Engerix-B followed by HBsAg/HVR1-1a VLPs, group 3 received only HBsAg/HVR1-1a VLPs and group 4 received HBsAg/HVR1-1a VLPs followed by Engerix-B (Table 2). One control animal (#15) was used which was not vaccinated.

### A. Immunisation of mice with and without pre-existing anti-HBs antibodies with HBsAQ/HVR1-1 a VLPs

Group 1 and group 2 mice were immunized with Engerix-B on days 0 and 10, and all eight mice developed antibodies by day 18 against HBsAg. Fig. 8A shows the results of an anti-HBs specific ELISA performed on serum samples taken on day 33; all samples were positive. The corresponding pre-immune serum samples were negative (data not shown). Mice which were not immunized with Engerix-B (group 3 and animal #15) did not develop anti-HBs.

Five anti-HBs positive mice (group 2) were immunized on days 24 and 61 with HBsAg/HVR1-1a VLPs. As a control and reference, three naive Balb/c mice without pre-existing anti-HBs (group 3) were also immunized with HBsAg/HVR1-1 a VLPs on days 24 and 61. Both group 2 and group 3 mice developed anti-HVR1-1a antibodies as determined in serum samples taken on day 75 (Fig. 8B). Serum samples derived from mouse #15 were not reactive with HBsAg particles or with HVR1-1a specific peptides (Fig 8). These results suggest that the VLPs containing the HVR1 epitope were immunogenic regardless of a pre-existing anti-HBs response and the optical density (OD) values (group 2 mice OD ~0.20 - ~0.6, group 3 mice OD -0.25 - -0.7) indicated that there were no substantial difference in the intensity of the anti-HVR1-1a antibody response (Fig. 8B).

Hence, serum samples from each mouse were collected at different time points and the antibody titers determined. The arithmetic mean of the titers at each time point was plotted as a function of time (Fig. 9 and Fig. 10). All mice immunized with Engerix-B (group 1 and group 2) developed anti-HBs with titers of 1:600 to 1:800 by day 18, Fig. 9A shows the data for the group 2 mice. On day 61 the arithmetic mean of the titer was 1:24000 and the lowest and highest titers were 1:6400 and 1:51200, respectively. The antibody titer persisted to day 199 which was the latest time point examined (Fig. 9A). Two of five mice in group 2 developed anti-HVR1-1a antibodies by day 61 (titers 1:300 and 1:800), and by day 75 all mice in this group developed anti-HVR1-1a antibodies; the lowest and highest titers were 1:200 and 1:3200, respectively (Fig. 9B). The decrease in the arithmetic mean of the anti-HVR1-1a antibody titer as indicated by the dashed line (Fig. 9B) between days 143 and 153 (1:2460 to 1:700) is due to the fact that the two animals with the highest anti-HVR1-1a antibody titers had to be sacrificed on day 143 because of an ophthalmia. The anti-HVR1-1a immune response induced by HBsAg/HVR1-1a VLPs persisted for at least another 100 days post immunization. Anti-HBs positive mice which did not receive HBsAg/HVR1-1a VLPs (group 1, Table 2) did not develop an anti-HVR1-1a antibody response (Fig. 8B, and data not shown).

Mice in group 3 were immunized with HBsAg/HVR1-1a VLPs only (Table 2), and the anti-HVR1-1a antibody titers in this group were compared with those in mice with pre-existing anti-HBs (group 2). Initially the anti-HBs response was measured in this group to evaluate if the modified particles could induce an anti-HBs immune response. Titration of serum samples taken at different time points showed that immunization with HBsAg/HVR1-1a VLPs induced antibodies directed against unmodified HBsAg-particles. Two of three mice developed anti-HBs antibodies (Fig. 10A) by day 75, in parallel with the anti-HVR1-1a antibodies (see below). The titer of the anti-HBs antibodies was 1:200 and 1:3200. Compared with mice (group 2) immunized with Engerix-B (Fig. 9A) or wildtype HBsAg-particles synthesized in HuH-7 cells (data not shown), the titers were low, or in some mice anti-HBs was not detectable.

Therefore, the insertion of 35aa into the 'a'-determinant region interferes with the immunogenicity of 'a'-determinant-specfic epitopes of the HBsAg protein. The assessment of the anti-HVR1-1a response in group 3 mice showed that, similar to group 2, one of the three mice developed an anti-HVR1-1a response by day 61 (1:50) and by day 75 all group 3 mice developed an immune response against HVR1-1a (Fig. 10B). The arithmetic mean of titers on day 75 was 1:3300 and the lowest and highest titers were 1:400 and 1:6400, respectively. These titers were also similar to anti-HVR1-1a antibody titers generated in the group 2 mice and correlate with the anti-HVR1-1a specific OD values determined for serum samples taken on day 75 (Fig. 8). Similar to the group 2 mice, the anti-HVR1-1a antibody response in the group 3 mice persisted at least for another 100 days (Fig. 10B).

### B. Immunization of mice with modified HBsAg VLPs followed by Engerix-B.

To confirm that vaccination with wildtype HBsAg particles and 'a'-determinant modified HBsAg particles do not mutually interfere, the effect of vaccination with HBsAg/HVR1-1a VLPs prior to vaccination with Engerix-B was assessed. Three mice in group 4 (Table 2) were injected with HBsAg/HVR1-1a VLPs and developed anti-HVR1-1a titers of 1:300, 1:6400, and 1:25600, respectively on day 153 (Fig. 11B). One mouse also developed an anti-HBs titer of 1:200 (day 61) to 1:600 (day 160) (Fig. 11A). The mice were then immunized with Engerix-B, on days 160, 172, and 185. All mice developed anti-HBs antibodies by day 185 with titers between 1:600 and 1:12800 that rose between 1:3200 and 1:25600 on day 199. Therefore, immunization with HBsAg/HVR1-1a VLPs did not interfere with the synthesis of antibodies to HBsAg after immunization with the standard HBV vaccine, Engerix-B.

The present invention includes within its scope modifications and adaptations apparent to one skilled in the art. Furthermore, throughout the specification, unless the context requires otherwise, the word "comprise" or variations such as "comprises" or "comprising", will be understood to imply the inclusion of a stated integer or group of integers but not the exclusion of any other integer or group of integers.
1. Berger, J., Hauber, J., Hauber, R., Geiger, R., and Cullen, B.R. (1988) Secreted alkaline phosphatase: a powerful new indicator of gene expression in eukaryotic cells. Gene 66, 1-10.
2. Chang, F.-L., Chen. P.-J., Tu, S.-J., Wang, C.-J., and Chen, D.-S. (1991). The large form of hepatitis delta antigen is crucial for assembly of hepatitis delta virus. Proc. Natl. Acad. Sci. USA 88, 8490-8494.
3. Dubois, M.F., Pourcel, C., Rousset, S., Chany, C., and Tiollais, P. (1980). Excretion of hepatitis B surface antigen particles from mouse cells transformed with cloned viral DNA. Proc. Natl. Acad. Sci USA 77, 4549-4553.
4. Graham, F.L., and van der Eb, A.J. (1973). A new technique for the assay of infectivity of human adenovirus 5 DNA. Virology 52, 456-467.
5. Harvey, T.J., Macnaughton, T.B., and Gowans, E.J. (1997). The development and characterisation of a SV40 T-antigen positive cell line of human hepatic origin. J. Virol. Methods 65, 67-74.
6. Kolykhalov, A.A., Agapov, E.V., Blight, K.J., Mihalik, K., Feinstone, S.M., and Rice, C.M. (1997) Transmission of hepatitis C by intrahepatic inoculation with transcribed RNA. Science 277, 570-574.
7. Moriarty, A.M., Hoyer, B.H., Shih, J.W., Gerin, J.L., and Hamer, D.H. (1981). Expression of the hepatitis B virus surface antigen gene in cell culture by using a simian virus 40 vector. PNAS USA 78, 2606-2610.
8. Nakabayashi, H., Taketa, K., Miyano, K., Yamane, T., and Sato, J. (1982). Growth of human hepatoma cell lines with differentiated functions in chemically defined medium. Cancer Res. 42, 3858-3863.
9. Trowbridge, R., and Gowans, E.J. (1998) Molecular cloning of an Australian isolate of hepatitis C virus. Arch. Virol. 143, 501-511.
10. Wang, C.-J., Chen, P.-J., Wu, J.-C., Patel, D., and Chen, D.-S. (1991). Small-form hepatitis B surface antigen is sufficient to help in the assembly of hepatitis delta virus-like particles. J. Virol. **65**, 6630-6636.

SEQUENCE LISTING

<110>  Monash University

<120>  Improved virus-like particles based on small envelope protein from
Hepatitis B (HBsAg-S)

<130>  P104604EP-PCT/JPD

<140>  01953691.1
<141>  2001-07-30

<160>  16

<170>  PatentIn version 3.1

<210>  1
<211>  29
<212>  DNA
<213>  Artificial

<220>
<223>  oligonucleotide

<400>  1
gatgaattct cactgcattc tagttgtgg                                          29


<210>  2
<211>  30
<212>  DNA
<213>  Artificial

<220>
<223>  oligonucleotide

<400>  2
gatgaattcc ttctgctcta aaccggatcg                                         30


<210>  3
<211>  30
<212>  DNA
<213>  Artificial

<220>
<223>  oligonucleotide

<400>  3
gactaccggt caaggaacct ctatgtatcc                                         30


<210>  4
<211>  31
<212>  DNA
<213>  Artificial

<220>
<223>  oligonucleotide

<400>  4
cttgaccggt agtcatgcag gtccggcatg g                                       31


<210>  5
<211>  21
<212>  DNA
<213>  Artificial

<220>
<223>  oligonucleotide

<400>  5
ccggtgggga cacccacacg a                                                21


<210>  6
<211>  21
<212>  DNA
<213>  Artificial

<220>
<223>  oligonucleotide

<400>  6
ccggtcgtgt gggtgtcccc a                                                21


<210>  7
<211>  66
<212>  DNA
<213>  Artificial

<220>
<223>  oligonucleotide

<400>  7
ccggtgggga cacccacacg acgggggggg tggcgggccg cgacacgctg cgcttcacgg      60

ggttca                                                                 66


<210>  8
<211>  66
<212>  DNA
<213>  Artificial

<220>
<223>  oligonucleotide

<400>  8
ccggtgaacc ccgtgaagcg cagcgtgtcg cggcccgcca ccccccccgt cgtgtgggtg      60

tcccca                                                                 66


<210>  9
<211>  30
<212>  DNA
<213>  Artificial

<220>
<223>  oligonucleotide

<400>  9
tgactaccgg tggtgtttac aagctggatc                                       30


<210>  10
<211>  28
<212>  DNA
<213>  Artificial

<220>
<223>  oligonucleotide

<400>  10
tgactaccgg tggggacacc cacacgac                                          28


<210>  11
<211>  32
<212>  DNA
<213>  Artificial

<220>
<223>  oligonucleotide

<400>  11
tgactaccgg tggggaaacc cacgtcaccg gg                                     32


<210>  12
<211>  27
<212>  DNA
<213>  Artificial

<220>
<223>  oligonucleotide

<400>  12
tgactaccgg tgttgatcag ttggatg                                           27


<210>  13
<211>  28
<212>  DNA
<213>  Artificial

<220>
<223>  oligonucleotide

<400>  13
gactaccggt gatggggtgg cagctggc                                          28


<210>  14
<211>  27
<212>  PRT
<213>  Hepatitis C virus

<400>  14

Glu Thr His Val Thr Gly Gly Ser Ala Gly Arg Thr Thr Ala Gly Leu
1               5                   10                  15


Val Gly Leu Leu Thr Pro Gly Ala Lys Gln Asn
            20                  25


<210>  15
<211>  27
<212>  PRT
<213>  Hepatitis C virus

<400>  15

Asp Thr His Thr Thr Gly Gly Val Ala Gly Arg Asp Thr Leu Arg Phe
1               5                   10                  15


Thr Gly Phe Phe Ser Phe Gly Pro Lys Gln Lys
            20                  25

<210>  16
<211>  18
<212>  PRT
<213>  Human papillomavirus

<400>  16

Asp Ser Thr Leu Arg Leu Cys Val Gln Ser Thr His Val Asp Ile Arg
1               5                   10                  15

Thr Leu

??

??

??

??

W:\Files\FF35396-03\FF35396-03_020305_sequence listing.txt

## Claims

1. An isolated polynucleotide comprising a HBsAg-S coding sequence that is adapted to receive an insert coding sequence, within a part of the HBsAg-S coding sequence that encodes an exposed site within the external loop of HBsAg-S, and still encode a HBsAg-S that is able to assemble into a VLP.

2. An isolated polynucleotide according to claim 1 wherein the HBsAg-S coding sequence encodes a HBsAg-S that can assemble into a secretion competent VLP.

3. An isolated polynucleotide according to claim 1 wherein the HBsAg-S coding sequence encodes a HBsAg-S that is retained within its host cell.

4. An isolated polynucleotide according to any one of claims 1 to 3 wherein the HBsAg-S coding sequence encodes a HBsAg-S that can assemble into a VLP that includes a disrupted native HBsAg epitope.

5. An isolated polynucleotide according to claim 1 wherein the HBsAg-S coding sequence is adapted to receive an insert coding sequence within the part of the HBsAg-S polynucleotide encoding:
(i) amino acids 114 to 160 or 169 of HBsAg-S;
(ii) amino acids 120 to 160 of HBsAg-S;
(iii) the 'a' determinant of HBsAg-S;
(iv) amino acids 120 to 150 of HBsAg-S;
(v) amino acids 124 to 147 of HBsAg-S;
(vi) amino acids 124-145, 124-142, 124-140, 124-138, 124-136, 124-134, 124-132, 124-130, 124-128 or 124-126 of HBsAg-S; or
(vii) amino acids 127 and 128 of HBsAg-S.

6. An isolated polynucleotide comprising a HBsAg-S coding sequence that is adapted to receive an insert coding sequence, between codons 127 and 128; and still encode a HBsAg-S that is able to assemble into a VLP.

7. An isolated polynucleotide comprising a HBsAg-S coding sequence, which defines a restriction site within a part of the HBsAg-S coding sequence that encodes an exposed site within the external loop of HBsAg-S, and still encodes a HBsAg-S that is able to assemble into a VLP.

8. An isolated polynucleotide according to claim 7 wherein the inclusion of the restriction site doesn't affect the amino acid sequence of the encoded HBsAg-S.

9. An isolated polynucleotide according to claim 7 or 8 wherein the restriction site is an Agel site between codon 127 and 128 of the HBsAg-S coding sequence.

10. An isolated polynucleotide according to claim 7, 8 or 9 wherein codon 127 in the native HBsAg-S coding sequence is changed from ACT to ACC and codon 128 is changed from GCT to GGT.

11. An isolated polynucleotide according to any one of the preceding claims adapted to receive an insert coding sequence of up to about 5 to 100 amino acids, about 10 to 90 amino acids, about 20 to 80 amino acids, about 30 to 70 amino acids, or about 40 to 60 amino acids and still encode a HBsAg-S that is able to assemble into a VLP.

12. An isolated polynucleotide according to any one of the preceding claims adapted to receive an insert coding sequence of up to about 35 or 60 amino acids.

13. An isolated polynucleotide according to any one of the preceding claims further comprising an insert coding sequence.

14. An isolated polynucleotide according to claim 13 wherein the insert coding sequence is of HBV origin.

15. An isolated polynucleotide according to claim 13 wherein the insert coding sequence is of heterologous origin.

16. An isolated polynucleotide according to claim 15 wherein the heterologous insert coding sequence is of bacterial, viral, animal or plant origin.

17. An isolated polynucleotide according to claim 13 wherein the insert coding sequence is of a HCV sequence.

18. An isolated polynucleotide according to any one of claims 13 to 17 wherein the insert coding sequence encodes (i) an immunological protein or portion thereof that include an epitope or (ii) a binding protein or portion thereof that encodes a binding domain.

19. An isolated polynucleotide according to claim 17 wherein the insert coding sequence encodes an antigenic portion of HVR1 sequence of the E2 protein.

20. An isolated polynucleotide according to any one of the preceding claims capable of expression to yield HBsAg-S that are able to assemble into a VLP upon which are presented the expression product of the insert coding sequence in a correct surface orientation.

21. An isolated polynucleotide according to any one of the preceding claims wherein the HBsAg-S coding sequence comprises a HBsAg-M coding sequence.

22. An isolated polynucleotide according to any one of the preceding claims wherein the HBsAg-S coding sequence comprises a HBsAg-L coding sequence.

23. A method for producing an isolated polynucleotide encoding a HBsAg-S coding sequence comprising the steps of (i) isolating the HBsAg-S coding sequence (ii) modifying the HBsAg-S coding sequence such that, it is adapted to receive an insert coding sequence within a part of the HBsAg-S coding sequence that encodes an exposed site within the external loop of HBsAg-S, and still encode a HBsAg-S that is able to assemble into a VLP.

24. A vector comprising a polynucleotide of any one of claims 1 to 22.

25. A host cell comprising a polynucleotide of any one of claims 1 to 22 or a vector of claim 24.

26. A host cell according to claim 24 adapted to produce secretion competent VLPs.

27. A host cell according to claim 24 or 25 of bacterial, fungal, insect or mammalian origin or a cancer cell.

28. A protein, polypeptide or peptides encoded by a polynucleotide according to any one of claims 1 to 22.

29. A protein, polypeptide or peptide according to claim 28 capable of self-assembly into a VLP.

30. A VLP comprising a protein, polypeptide or peptide of claim 28 or 29.

31. A VLP according to claim 30 comprising HBsAg-S and an insert located within the exposed site within the external loop of HBsAg-S.

32. A VLP according to claim 30 or 31 comprising HBsAg-S and an insert within:
(i) amino acids 114 to 160 or 169 of HBsAg-S;
(ii) amino acids 120 to 160 of HBsAg-S;
(iii) the 'a' determinant of HBsAg-S;
(iv) amino acids 120 to 150 of HBsAg-S;
(v) amino acids 124 to 147 of HBsAg-S;
(vi) amino acids 124-145, 124-142, 124-140, 124-138, 124-136, 124-134, 124-132, 124-130, 124-128 or 124-126 of HBsAg-S; or
(vii) amino acids 127 and 128 of HBsAg-S.

33. A VLP according to any one of claims 30 to 32 further comprising a heterologous insert.

34. A VLP according to claim 33 wherein the insert is of bacterial viral, animal or plant origin.

35. A VLP according to claim 33 wherein the insert is a HCV protein.

36. A VLP according to any one of claims 33 to 35 sequences wherein the insert is an (i) immunological protein or portion thereof that includes an epitope or (ii) a binding protein or portion thereof that encodes a binding domain.

37. A method of producing a VLP comprising the steps of: (i) transfecting a cell with a vector encoding a HBsAg-S and an insert that upon expression is capable of assembling into the VLP; (ii) culturing said cell under conditions that enable the expression of the HBsAg-S including the insert and assembly of the VLP; and (iii) isolating the VLP.

38. A pharmaceutical composition comprising a VLP according to any one of claims 33 to 36 and physiologically acceptable carrier.

39. A method of generating an immune response in a patient comprising the step of administering to said patient an effective amount of a VLP according to any one of claims 33 to 36 or a pharmaceutical composition according to claim 38 wherein said VLP includes an immunogenic insert.

40. A method of generating an immune response in a patient comprising the steps of (i) administering to said patient a HBV immunogenic preparation and (ii) administering to said patient an effective amount of a VLP according to any one of claims 33 to 36 or a pharmaceutical composition according to claim 38 wherein said VLP includes an immunogenic insert.

41. An immunogenic preparation comprising a VLP according to any one of claims 33 to 36 or a pharmaceutical composition according to claim 38.

42. A VLP according to any one of claims 33 to 36 or a pharmaceutical composition according to claim 38 wherein the VLP is a hybrid VLP comprising a plurality of heterologous inserts.

43. A VLP composition comprising a plurality of VLPs according to any one of claims 33 to 36 and wherein each VLP comprises a single heterologous insert.

44. A VLP composition comprising a plurality of VLPs according to any one of claims 33 to 36 and wherein each VLP is a hybrid VLP comprising a plurality of heterologous inserts.

45. A method for treating a disease or disorder in a patient comprising administering to said patient an effective amount of a VLP according to any one of claims 33 to 36 or a composition of any one of claims 38 or 42 to 44.

46. The use of a VLP according to any one of claims 33 to 36 or a composition according to any one of claims 38 or 42 to 44 to deliver an agent to a target cell.

47. A method of producing a VLP adapted to deliver an agent to a target cell comprising the steps of: (i) transfecting a cell with a vector encoding a HBsAg-S and an insert that upon expression is capable of assembling into the VLP wherein said insert encodes said agent and a binding agent specific for said target cell; (ii) culturing said cell under conditions that enable the expression of the HBsAg-S including the insert and assembly of the VLP; and (iii) isolating the VLP.

48. A method of delivering an agent to a target cell comprising the steps of (i) preparing a VLP which presents a binding agent for the target cell and (ii) contacting the VLP with the media containing the target cell.
